# EUROPÄISCHE PATENTSCHRIFT

(11) **EP 2 653 396 B1**
(45) Veröffentlichungstag und Bekanntmachung des Hinweises auf die Patenterteilung: **25.10.2017**
(21) Anmeldenummer: 13160087.6
(22) Anmeldetag: 20.03.2013
(51) Int. Cl.: B65B 55/06, B65B 55/10, A61L 2/22, A61L 2/07, A61L 2/18, A61L 2/20, B67B 3/00

(54) **Sterilisation von Verpackungsbehältern**
Sterilization of packaging containers
Stérilisation de récipients d'emballage

(30) Priorität: 18.04.2012 DE 102012206389
(43) Veröffentlichungstag der Anmeldung: 23.10.2013
(73) Patentinhaber: Krones AG, 93073 Neutraubling (DE)
(72) Erfinder: Morawe, Thomas, 93073 Neutraubling (DE); Müller, Holger, 93073 Neutraubling (DE)
(74) Vertreter: Grünecker Patent- und Rechtsanwälte PartG mbB

(56) Entgegenhaltungen:
- EP-B1- 1 144 016
- US-A1- 2009 071 104

## Beschreibung

### Gebiet der Erfindung

Die vorliegende Erfindung betrifft ein Verfahren zur Sterilisation von Verpackungsbehältern vor der Abfüllung und insbesondere ein Verfahren zur Sterilisation von PET-Flaschen.

### Hintergrund der Erfindung

Bei der Abfüllung von flüssigen Lebensmitteln, beispielsweise von Fruchtsäften, in Verpackungsbehälter, insbesondere Kunststoffbehälter, beispielsweise Flaschen aus Polyethylenterephthalat (PET), muss eine große Reinlichkeit beachtet werden. Insbesondere ist es erforderlich, die Verpackungsbehälter außen und vor allem innen vor dem Abfüllen zu Sterilisieren. Die Sterilisation erfolgt häufig mithilfe eines Gemisches aus Wasserdampf und einem Desinfektionsmittel. So lässt sich beispielsweise mithilfe einer Zweistoffdüse ein Dampfgemisch aus Wasserdampf und Peressigsäure in das Innere der Verpackungsbehälter einführen, wo es an den Innenwänden niederschlägt. An der Außenseite der Verpackungsbehälter kann eine Sterilisation mithilfe temperierter Peressigsäure erfolgen.

Der Sterilisationsvorgang ist umso wirksamer, desto höhere Temperaturen insbesondere bei der Sterilisation des Behälterinneren mithilfe des Gemisches aus Wasserdampf und Desinfektionsmittel vorliegen. Höhere Sterilisationstemperaturen führen jedoch zu höheren unerwünschten Verformungen der Verpackungsbehälter. Dieses Problem stellt sich vor allem bei der Behandlung immer dünner werdender PET-Flaschen, die sich am Markt einer sehr großen Beliebtheit erfreuen.

Um die Verformung (den Schrumpf) während der Sterilisation des Behälterinneren in einem akzeptablen Bereich zu halten, wird im Stand der Technik während der Beaufschlagung des Behälterinneren mit dem Gemisch aus Wasserdampf und Desinfektionsmittel das Äußere des Behälters gleichzeitig mit einem kälteren Medium gekühlt. Insbesondere ist es im Stand der Technik bekannt, während der Sterilisation des Behälterinneren mit einem Gemisch aus Wasserdampf und Desinfektionsmittel bei über 95 °C eine Desinfektion der Außenseite des Behälters mithilfe einer lediglich auf 40 °C bis 60° C vorgewärmten Peressigsäurelösung vorzunehmen. Aufgrund des Temperaturunterschieds zwischen der für die Außendesinfektion verwendeten Peressigsäurelösung und des zur Innendesinfektion verwendeten Dampfgemischs kommt es zu einem auf die durch das Dampfgemisch aufgeheizten Wände des Behälters wirkenden Kühleffekt, wodurch dem Verformen (Schrumpfen) des Behälters entgegengewirkt werden kann. Andererseits ist eine Erwärmung der Peressigsäurelösung auf 40 °C bis 60° C nötig, um die gewünschte Sterilisation für die Außenseiten der Behälter zu erreichen. Der Kühleffekt ist somit durch den für die Entkeimung erforderlichen vorgegeben Temperaturbereich beschränkt und reicht bei sehr hohen Temperaturen des Gemisches aus Wasserdampf und Desinfektionsmittel von bis zu etwa 99 °C nicht dazu aus, dem Schrumpfen hinreichend vorzubeugen.

Es liegt somit der vorliegenden Erfindung die Aufgabe zugrunde, eine Sterilisation des Inneren von abzufüllenden Verpackungsbehältern, insbesondere PET-Flaschen, ohne signifikantes Schrumpfen der Behälter auch bei hohen Temperaturen eines zur Innensterilisation verwendeten Gemischs aus Wasserdampf und Desinfektionsmittel zu ermöglichen.

### Beschreibung der Erfindung

Die oben genannte Aufgabe wird durch das Verfahren gemäß Anspruch 1 gelöst. Das beanspruchte Verfahren zur Sterilisation von Verpackungsbehältern weist die Schritte auf:
Beaufschlagen des Inneren eines Verpackungsbehälters mit einem Gemisch aus Wasserdampf und einem Desinfektionsmittel, beispielsweise Peressigsäure, mit einer Temperatur von mindestens 90 °C, insbesondere mindestens 95 °C; und
gleichzeitig Beaufschlagen des Äußeren des Verpackungsbehälters mit einem Mittel, das ein Desinfektionsmittel umfasst, bei einer Temperatur von unterhalb von 40 °C, insbesondere mit einer Temperatur zwischen 20°C und 30° C.

Bei den Verpackungsbehältern kann es sich um Kunststoffflaschen handeln. Insbesondere kann das Verfahren zur Sterilisation von PET-Flaschen dienen. Dadurch dass während der Sterilisation des Inneren der Verpackungsbehälter mit einem Mittel, das ein Desinfektionsmittel umfasst, mit einem im Vergleich zum Stand der Technik deutlich geringeren Temperatur das Äußere der Verpackungsbehälter beaufschlagt wird, kann auch bei sehr hohen Temperaturen von über 95 °C, beispielsweise 99 °C, des Gemisches aus Wasserdampf und einem Desinfektionsmittel eine Verformung der Verpackungsbehälter effektiv verhindert werden.

Das Mittel kann eine Desinfektionsmittellösung, beispielsweise eine Peressiglösung, sein, und das Verfahren kann weiterhin den Schritt des Beaufschlagens des Äußeren des Verpackungsbehälters mit dem Mittel mit einer Temperatur von mindestens 40 °C, insbesondere mit einer Temperatur zwischen 40°C und 60° C, insbesondere nach Beenden des Schritts des Beaufschlagens des Inneren des Verpackungsbehälters mit dem Gemisch aus Wasserdampf und dem Desinfektionsmittel, umfassen. In dieser Weiterbildung ist also vorgesehen, während der Innensterilisation mit einer solchen Desinfektionsmittellösung von außen zu kühlen, die in einem weiteren Verfahrensschritt bei höherer Temperatur zur Sterilisation der Außenwände der Verpackungsbehälter verwendet wird.

Hierbei ist es vorteilhaft, das Mittel in einem Kreislauf zu führen, in dem das Mittel vor dem Beaufschlagen des Äußeren des Verpackungsbehälters mit dem Mittel mit einer Temperatur von mindestens 40 °C erhitzt wird und vor dem Beaufschlagen des Äußeren des Verpackungsbehälters mit dem Mittel bei einer Temperatur von unterhalb von 40 °C (während der Innensterilisation) gekühlt wird (s. auch detaillierte Beschreibung unten). Das Mittel kann hierbei sowohl nach dem Beaufschlagen des Äußeren der Verpackungsbehälter bei niedrigen Temperaturen (beispielsweise 20 °C bis 30 °C) zur Kühlung der Verpackungsbehälter während der Innensterilisation als auch nach Beaufschlagung des Äußeren der Verpackungsbehälter bei relativ hohen Temperaturen (beispielsweise 40 °C bis 60 °C) zur Außensterilisation gesammelt und dem Kreislauf wieder zugeführt werden.

Die Kühlung des Mittels vor dem Beaufschlagen mit einer Temperatur unterhalb von 40 °C kann in einem Wärmeübertrager mithilfe eines Kühlmediums erfolgen. Prinzipiell kann jedes geeignete Kühlmedium Verwendung finden. Beispielsweise kann Prozesswasser, Eiswasser, Glykol, oder Kühlturmwasser als Kühlmedium verwendet werden. Insbesondere kann jedoch Wasser als Kühlmedium verwendet werden, das zum Reinigen der Verpackungsbehälter nach der Sterilisation benutzt wird. In einer Weiterbildung umfasst das erfindungsgemäße Verfahren das Reinigen des Verpackungsbehälters mit Wasser durch einen Rinser nach dem Beaufschlagen des Äußeren des Verpackungsbehälters mit dem Mittel mit einer Temperatur von mindestens 40 °C und das Sammeln des zur Reinigung verwendeten Wassers nach der Reinigung. Hierbei wird dann das Mittel vor dem Beaufschlagen des Äußeren des Verpackungsbehälters mit dem Mittel bei einer Temperatur von unterhalb von 40 °C in einem Wärmeübertrager mithilfe des gesammelten Wassers als Kühlmedium gekühlt.

Bei einer separaten Sterilisation von Verpackungsbehälterverschlüssen kann das Verfahren die Schritte des Sterilisierens des Verschlusses des Verpackungsbehälters und des nachfolgenden Reinigens des Verschlusses des Verpackungsbehälters mit Wasser, insbesondere Sterilwasser, sowie des Sammelns des zur Reinigung des Verschlusses des Verpackungsbehälters verwendeten Wassers nach der Reinigung umfassen, wobei dann das Mittel vor dem Beaufschlagen des Äußeren des Verpackungsbehälters mit dem Mittel bei einer Temperatur von unterhalb von 40 °C in einem Wärmeübertrager mithilfe des gesammelten Wassers als Kühlmedium gekühlt wird.

Somit kann also das jeweilige Rinswasser, das im Stand der Technik verworfen wird, noch weiter genutzt werden.

Die oben genannte Aufgabe wird auch gelöst durch eine Vorrichtung zum Behandeln von Verpackungsbehältern, insbesondere PET-Flaschen, mit
einem ersten Rotor mit Aufnahmeplätzen für die Verpackungsbehälter;
einer ersten Einrichtung, die zum Beaufschlagen des Inneren der Verpackungsbehälter an den Aufnahmeplätzen mit einem Gemisch aus Wasserdampf und einem Desinfektionsmittel mit einer Temperatur von mindestens 90 °C, insbesondere mindestens 95 °C, ausgebildet ist; und
einer zweiten Einrichtung, die dazu ausgebildet ist, gleichzeitig mit dem Beaufschlagen des Inneren der Verpackungsbehälter das Äußere der Verpackungsbehälter mit einem Mittel (zur Kühlung während der Innensterilisation) bei einer Temperatur von unterhalb von 40 °C, insbesondere mit einer Temperatur zwischen 20°C und 30° C, zu beaufschlagen.

Die Vorrichtung kann einen zweiten Rotor mit Aufnahmeplätzen für die Verpackungsbehälter, der dem ersten Rotor nachgeordnet ist, und eine dritte Einrichtung, die zum Beaufschlagen des Äußeren der Verpackungsbehälter mit dem Mittel, das ein Desinfektionsmittel umfasst, (nunmehr zu Sterilisation) mit einer Temperatur von mindestens 40 °C, insbesondere mit einer Temperatur zwischen 40°C und 60° C, ausgebildet ist, umfassen. Ein Rotor in Form eines Drehtisches und Drehachse, wie er für den ersten und zweiten Rotor verwendet werden kann, ist beispielsweise in EP 1 354 799 B1 beschrieben. Der erste und der zweite Rotor können am Umfang Greifzangen zum Halten von Flaschen an/unterhalb deren Halskragen in aufrechter Position mit der Mündung nach oben zeigend aufweisen. Insbesondere kann der erste Rotor Mischdüsen zur Innensterilisation aufweisen, und es kann der zweite Rotor (Misch-)Düsen zum Aufbringen des Mittels, das ein Desinfektionsmittel umfasst, seitlich an der Bewegungsbahn der Verpackungsbehälter angeordnet aufweisen.

Die Vorrichtung kann zudem mit einem ersten Wärmeübertrager ausgestattet sein, der dazu ausgebildet ist, das Mittel zu erhitzen, bevor es der dritten Einrichtung zum Beaufschlagen des Äußeren der Verpackungsbehälter zugeführt wird. Die Vorrichtung kann zudem mit einem zweiten Wärmeübertrager ausgestattet sein, der dazu ausgebildet ist, das Mittel zu kühlen, bevor es der zweiten Einrichtung zum Beaufschlagen des Äußeren der Verpackungsbehälter zugeführt wird. Hierbei kann der zweite Wärmeübertrager als Kühlmedium Prozesswasser, Eiswasser, Glykol, oder Kühlturmwasser verwenden.

Die Vorrichtung kann einen ersten Rinser aufweisen, der dem zweiten Rotor nachgeordnet ist, und der dazu ausgebildet ist, die Verpackungsbehälter mit Wasser, insbesondere Sterilwasser, zu reinigen, und sie kann eine erste Sammeleinrichtung aufweisen, die dazu ausgebildet ist, das zur Reinigung verwendete Wasser nach dem Reinigen zu sammeln und dem zweiten Wärmeübertrager als Kühlmedium zuzuführen.

Gemäß einer Weiterbildung umfasst die Vorrichtung zum Behandeln von Verpackungsbehältern eine Einrichtung, die dazu ausgebildet ist, die Verschlüsse der Verpackungsbehälter zu sterilisieren, einen zweiten Rinser, der der Einrichtung, die dazu ausgebildet ist, die Verschlüsse der Verpackungsbehälter zu reinigen, nachgeordnet ist, und der dazu ausgebildet ist, die Verschlüsse der Verpackungsbehälter mit Wasser zu reinigen, und eine zweite Sammeleinrichtung, die dazu ausgebildet ist, das zur Reinigung der Verschlüsse der Verpackungsbehälter verwendete Wasser nach dem Reinigen zu sammeln und dem zweiten Wärmeübertrager als Kühlmedium zuzuführen.

Im Folgenden werden Ausführungsformen eines erfindungsgemäßen Verfahrens unter Bezugnahme auf die Zeichnung beschrieben. Die beschriebenen Ausführungsformen sind in jeder Hinsicht lediglich als illustrativ und nicht als einschränkend anzusehen und verschiedene Kombinationen der angeführten Merkmale sind in der Erfindung eingeschlossen.
Figur 1 veranschaulicht ein Beispiel für das erfindungsgemäße Verfahren zur Sterilisation von Verpackungsbehältern in Form eines Flussdiagramms.
Figur 2 stellt eine Prinzipskizze für die Anordnung zweier Wärmeübertrager zur Kühlung bzw. Erhitzung eines Mittels, das erfindungsgemäß zur Beaufschlagung des Äußeren der Verpackungsbehälter verwendet wird dar.
Figur 3 veranschaulicht in einer Prinzipskizze einen Kühlkreislauf, der für die Kühlung einer Desinfektionsmittellösung vor Beaufschlagung des Äußeren von Verpackungsbehältern während der Innensterilisation gemäß einem erfindungsgemäßen Beispiel Verwendung finden kann.

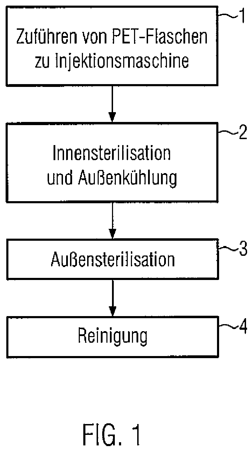

Figur 1 veranschaulicht Schritte eines Sterilisationsverfahrens für abzufüllende PET-Flaschen. Die PET-Flaschen werden, beispielsweise über eine Endlosförderer, einer Injektionsmaschine mit, beispielsweise vergleichbar einer solchen, wie sie in EP 1 144 016 B1 beschrieben ist, zugeführt 1. Die Injektionsmaschine weist einen Rotor mit Aufnahmeplätzen für die PET-Flaschen auf. Auf dem Drehtisch des Rotors sind Mischdüsen zum Einblasen eines Gemischs aus Wasserdampf und einem Desinfektionsmittel in das Innere der PET-Flaschen zur Innensterilisation 2 angebracht. Die Mischdüsen können Zwei-Komponenten-Zerstäubungsdüsen sein. Gleichzeitig mit der Innensterilisation erfolgt eine Kühlung der Außenwände der PET-Flaschen 2. Um eine hochgradige Sterilisation zu erreichen, wird das Gemisch aus Wasserdampf und einem Desinfektionsmittel mit sehr hohen Temperaturen, insbesondere oberhalb von 95 °C, in das Innere der PET-Flaschen geblasen, wo es an den Innenwänden kondensiert. Um eine Schrumpfung der PET-Flaschen während der Innensterilisation 2 zu vermeiden, erfolgt eine Kühlung 2 mit einem Mittel, das ein Desinfektionsmittel umfasst, mit einer Temperatur unterhalb von 40 °C, insbesondere mit einer Temperatur zwischen 20 °C und 30 °C.

Nach Beendigung der Innensterilisation werden die PET-Flaschen zu einem weiteren Rotor zur Außensterilisation 3 geliefert. Die Außensterilisation 3 erfolgt mit dem gleichen Mittel, das zur Kühlung während der Innensterilisation verwendet wurde, jedoch bei höheren Temperaturen, die zur Keimtötung erforderlich sind. Das Desinfektionsmittel kann Peressigsäure sein oder umfassen, und das Mittel kann eine Peressigsäurelösung sein. Das Desinfektionsmittel und das Mittel können auch Wasserstoffperoxid und zusätzlich ein Tensid aufweisen.

Es kann der Prozessschritt 2 zur gleichzeitigen Innensterilisation und Außenkühlung nach dem Prozessschritt 3 zur Außensterilisation noch einmal wiederholt werden, indem die PET-Flaschen beispielsweise ein weiteres Mal dem ersten Rotor zugeführt werden, wie es in EP 1 144 016 B1 beschrieben ist.

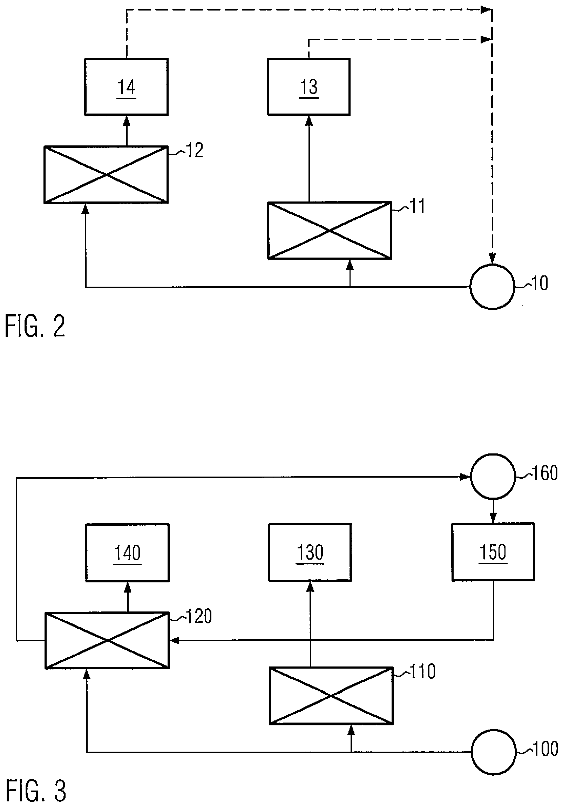

Nach erfolgter Sterilisation 2, 3 werden die PET-Flaschen in einem Rinser gereinigt 4. Das verwendete Reinigungsmedium kann in diesem Beispiel ein steriles Medium, beispielsweise Sterilwasser, sein. Das Mittel wird zur Beaufschlagung während der Innensterilisation auf eine relativ niedrige Temperatur, beispielsweise zwischen 20 °C und 30 °C, gekühlt und zur Beaufschlagung zur Außensterilisation auf eine relativ hohe Temperatur, beispielsweise zwischen 40 °C und 60 °C, erhitzt (s. auch Figur 2). Die Kühlung kann in einem Wärmeübertrager erfolgen, dem als Kühlmedium nach dem Reinigen 4 gesammeltes Rinswasser zugeführt wird.

Figur 2 zeigt Elemente, die in einem Beispiel der erfindungsgemäßen Vorrichtung Verwendung finden. Eine Desinfektionsmittellösung wird von einer Quelle 10 an einen ersten Wärmeübertrager 11 und einen zweiten Wärmeübertrager 12 geliefert. Die Desinfektionsmittellösung kann beispielsweise ein in Wasser gelöstes Tensid und 4000 ppm Wasserstoffperoxid und 2500 ppm Peressigsäure aufweisen. In dem ersten Wärmeübertrager 11 wird die Desinfektionsmittellösung erhitzt und dann zu einer Einrichtung 13 zur Beaufschlagung des Äußeren von Verpackungsbehältern zur Außensterilisation derselben geliefert.

In dem zweiten Wärmeübertrager 12 wird die Desinfektionsmittellösung gekühlt und dann zu einer Einrichtung 14 zur Beaufschlagung des Inneren der Verpackungsbehältern zur Innensterilisation derselben geliefert. In dieser Einrichtung 14 erfolgt eine Sterilisation des Inneren der Flachen mit einem heißen Gemisch aus Wasserdampf und einem Desinfektionsmittel (bei Temperaturen oberhalb von 95 °C) und eine gleichzeitige Kühlung des Äußeren der Flaschen durch die durch den zweiten Wärmeübertrager 12 gekühlte Desinfektionsmittellösung. In dem ersten Wärmeübertrager 11 wird die Desinfektionsmittellösung beispielsweise auf eine Temperatur zwischen 40 °C und 60 °C erhitzt, während sie in dem zweiten Wärmeübertrager 12 beispielsweise auf eine Temperatur zwischen 20 °C und 30 °C gekühlt wird.

Die in der Einrichtung 13 zur Beaufschlagung des Äußeren von Verpackungsbehältern zur Außensterilisation nach dem Beaufschlagen gesammelte Desinfektionsmittellösung und die in der Einrichtung 14 zur Beaufschlagung des Inneren der Verpackungsbehältern zur Innensterilisation nach dem Beaufschlagen gesammelte Desinfektionsmittellösung kann der Quelle 10 bzw. einer Lieferleitung stromabwärts der Quelle wieder zugeführt werden. Es kann die gesammelte Desinfektionsmittellösung gegebenenfalls durch Aufbereitungseinrichtungen (nicht gezeigt), wie beispielsweise Filtereinheiten und/oder durch Dosiereinrichtungen für Desinfektionsmittelkonzentrat, wieder in einen für die Wiederverwendung geeigneten Grundzustand gebracht werden, bevor sie der Quelle 10 wieder zugeführt wird.

Die Kühlung der Desinfektionsmittellösung in dem zweiten Wärmeübertrager 12 erfolgt mithilfe eines Kühlmediums. Figur 3 zeigt einen ähnlichen Aufbau wie denjenigen, der in Figur 2 dargestellt ist. Eine Quelle 100 liefert eine Desinfektionsmittellösung an einen ersten 110 und zweiten 120 Wärmeübertrager. Die Desinfektionsmittellösung wird in dem ersten Wärmeübertrager 110 erhitzt und einer Einrichtung 130 zur Beaufschlagung des Äußeren von Verpackungsbehältern zur Außensterilisation derselben zugeführt. In dem zweiten Wärmeübertrager 120 wird die Desinfektionsmittellösung gekühlt und dann zu einer Einrichtung 140 zur Beaufschlagung des Inneren der Verpackungsbehältern zur Innensterilisation derselben geliefert. Die sterilisierten Verpackungsbehälter werden nach Verlassen der Einrichtung 130 zur Beaufschlagung des Äußeren von Verpackungsbehältern einem Rinser 150 zur Reinigung zugeführt. Die Reinigung erfolgt mit Wasser, dem gegebenenfalls ein Reinigungsmittel zugegeben ist, das von einer Quelle 160 stammt. Das beim Reinigen verbrauchte Rinswasser wird dem zweiten Wärmeübertrager 120 als Kühlmedium zum Kühlen der von der Quelle 100 gelieferten Desinfektionsmittellösung zugeführt. Nach Wärmeaustausch in dem zweiten Wärmeübertrager 120 wird das Rinswasser der Quelle 160 im Rahmen eines Kreislaufes wieder zugeführt, wodurch, das Rinswasser also, anders als im Stand der Technik bekannt, nicht verworfen wird, sondern eine weitere Verwendung im Prozess findet. Hierzu wird das gesammelte Rinswasser vor der Quelle 160 durch geeignete Aufbereitungsmittel (nicht gezeigt), wie beispielsweise Sterilfiltereinheiten, wieder in einen für die Wiederverwendung geeigneten Zustand gebracht. Alternativ ist die Wiederverwendung in einem anderen Prozessschritt, beispielsweise zur Vorreinigung von Teilen der Vorrichtung zum Behandeln von Verpackungsbehältern oder zum Ausmischen von Reinigungslösungen zur Reinigung von Teilen der Vorrichtung zum Behandeln von Verpackungsbehältern, möglich. Auch in diesem alternativen Beispielen wird das Rinswasser, anders als im Stand der Technik bekannt, nicht verworfen.

## Patentansprüche

1. Verfahren zur Sterilisation von Verpackungsbehältern mit den Schritten:
Beaufschlagen des Inneren eines Verpackungsbehälters mit einem Gemisch aus Wasserdampf und einem Desinfektionsmittel mit einer Temperatur von mindestens 90 °C, insbesondere mindestens 95 °C; und
gleichzeitig Beaufschlagen des Äußeren des Verpackungsbehälters mit einem Mittel, das ein Desinfektionsmittel umfasst, bei einer Temperatur von unterhalb von 40 °C, insbesondere mit einer Temperatur zwischen 20°C und 30° C.

2. Das Verfahren gemäß Anspruch 1, in dem das Mittel eine Desinfektionsmittellösung ist und weiterhin den Schritt umfassend: Beaufschlagen des Äußeren des Verpackungsbehälters mit dem Mittel mit einer Temperatur von mindestens 40 °C, insbesondere mit einer Temperatur zwischen 40°C und 60° C, insbesondere nach Beenden des Schritts des Beaufschlagens des Inneren des Verpackungsbehälters mit dem Gemisch aus Wasserdampf und dem Desinfektionsmittel.

3. Das Verfahren gemäß Anspruch 2, in dem das Mittel in einem Kreislauf geführt wird, in dem das Mittel
a) vor dem Beaufschlagen des Äußeren des Verpackungsbehälters mit dem Mittel mit einer Temperatur von mindestens 40 °C erhitzt wird; und
b) vor dem Beaufschlagen des Äußeren des Verpackungsbehälters mit dem Mittel bei einer Temperatur von unterhalb von 40 °C gekühlt wird.

4. Das Verfahren gemäß Anspruch 3, weiterhin mit den Schritten:
Reinigen des Verpackungsbehälters mit Wasser, insbesondere Sterilwasser, durch einen Rinser nach dem Beaufschlagen des Äußeren des Verpackungsbehälters mit dem Mittel mit einer Temperatur von mindestens 40 °C, und
Sammeln des zur Reinigung verwendeten Wassers nach der Reinigung;
und wobei das Mittel vor dem Beaufschlagen des Äußeren des Verpackungsbehälters mit dem Mittel bei einer Temperatur von unterhalb von 40 °C in einem Wärmeübertrager mithilfe des gesammelten Wassers als Kühlmedium gekühlt wird.

5. Das Verfahren gemäß Anspruch 3 oder 4, weiterhin mit den Schritten:
Sterilisieren des Verschlusses des Verpackungsbehälters;
nachfolgend Reinigen des Verschlusses des Verpackungsbehälters mit Wasser, insbesondere Sterilwasser; und
Sammeln des zur Reinigung des Verschlusses des Verpackungsbehälters verwendeten Wassers nach der Reinigung;
und wobei
das Mittel vor dem Beaufschlagen des Äußeren des Verpackungsbehälters mit dem Mittel bei einer Temperatur von unterhalb von 40 °C in einem Wärmeübertrager mithilfe des gesammelten Wassers als Kühlmedium gekühlt wird.

6. Das Verfahren gemäß einem der vorhergehenden Ansprüche, in dem das Desfinfektionsmittel Peressigsäure ist und/oder das Mittel eine Peressigsäurelösung ist.

7. Das Verfahren gemäß einem der vorhergehenden Ansprüche, in dem der Verpackungsbehälter eine PET-Flasche ist.

8. Vorrichtung zum Behandeln von Verpackungsbehältern, insbesondere PET-Flaschen, mit
einem ersten Rotor mit Aufnahmeplätzen für die Verpackungsbehälter;
einer ersten Einrichtung, die zum Beaufschlagen des Inneren der Verpackungsbehälter an den Aufnahmeplätzen mit einem Gemisch aus Wasserdampf und einem Desinfektionsmittel mit einer Temperatur von mindestens 90 °C, insbesondere mindestens 95 °C, ausgebildet ist; und
einer zweiten Einrichtung, die dazu ausgebildet ist, gleichzeitig mit dem Beaufschlagen des Inneren der Verpackungsbehälter das Äußere der Verpackungsbehälter mit einem Mittel, das ein Desinfektionsmittel umfasst, bei einer Temperatur von unterhalb von 40 °C, insbesondere mit einer Temperatur zwischen 20°C und 30°C, zu beaufschlagen.

9. Die Vorrichtung gemäß Anspruch 8, weiterhin mit
einem zweiten Rotor mit Aufnahmeplätzen für die Verpackungsbehälter, der dem ersten Rotor nachgeordnet ist,
einer dritten Einrichtung, die zum Beaufschlagen des Äußeren der Verpackungsbehälter mit dem Mittel mit einer Temperatur von mindestens 40 °C, insbesondere mit einer Temperatur zwischen 40°C und 60° C, ausgebildet ist.

10. Die Vorrichtung gemäß Anspruch 9, weiterhin mit einem ersten Wärmeübertrager, der dazu ausgebildet ist, das Mittel zu erhitzen, bevor es der dritten Einrichtung zum Beaufschlagen des Äußeren der Verpackungsbehälter zugeführt wird.

11. Die Vorrichtung gemäß einem der Ansprüche 8 bis 10, weiterhin mit einem zweiten Wärmeübertrager, der dazu ausgebildet ist, das Mittel zu kühlen, bevor es der zweiten Einrichtung zum Beaufschlagen des Äußeren der Verpackungsbehälter zugeführt wird.

12. Die Vorrichtung gemäß einem der Ansprüche 8 bis 11, weiterhin mit einem ersten Rinser, der dem zweiten Rotor nachgeordnet ist, und der dazu ausgebildet ist, die Verpackungsbehälter mit Wasser, insbesondere Sterilwasser, zu reinigen, und mit einer ersten Sammeleinrichtung, die dazu ausgebildet ist, das zur Reinigung verwendete Wasser nach dem Reinigen zu sammeln und dem zweiten Wärmeübertrager als Kühlmedium zuzuführen.

13. Die Vorrichtung gemäß einem der Ansprüche 8 bis 12, weiterhin mit einer Einrichtung, die dazu ausgebildet ist, die Verschlüsse der Verpackungsbehälter zu sterilisieren;
einem zweiten Rinser, der der Einrichtung, die dazu ausgebildet ist, die Verschlüsse der Verpackungsbehälter zu sterilisieren, nachgeordnet ist, und der dazu ausgebildet ist, die Verschlüsse der Verpackungsbehälter mit Wasser, insbesondere Sterilwasser, zu reinigen, und
einer zweiten Sammeleinrichtung, die dazu ausgebildet ist, das zur Reinigung verwendete Wasser nach dem Reinigen zu sammeln und dem zweiten Wärmeübertrager als Kühlmedium zuzuführen.

## Claims

1. Method for sterilizing packaging containers, comprising the steps of:
treating the interior of a packaging container with a mixture consisting of water vapor and a disinfectant with a temperature of at least 90°C, particularly at least 95°C; and
simultaneously treating the exterior of the packaging container with an agent comprising a disinfectant, at a temperature below 40°C, particularly with a temperature between 20°C and 30°C.

2. Method according to claim 1, in which the agent is a disinfectant solution, and further comprising the step of: treating the exterior of the packaging container with the agent with a temperature of at least 40°C, particularly with a temperature between 40°C and 60°C, particularly after completion of the step of treating the interior of the packaging container with the mixture consisting of water vapor and the disinfectant.

3. Method according to claim 2, in which the agent is circulated in that the agent
a) is heated before the treatment of the exterior of the packaging container with the agent with a temperature of at least 40°C, and
b) is cooled before the treatment of the exterior of the packaging container with the agent at a temperature of less than 40°C.

4. Method according to claim 3, further comprising the steps of:
cleaning the packaging container with water, particularly sterile water, by a rinser after treatment of the exterior of the packaging container with the agent with a temperature of at least 40°C, and
collecting the water used for cleaning after the cleaning process;
and wherein before the treatment of the exterior of the packaging container with the agent at a temperature of less than 40°C the agent is cooled in a heat exchanger with the help of the collected water as cooling medium.

5. Method according to claim 3 or 4, further comprising the steps of:
sterilizing the closure of the packaging container;
subsequently cleaning the closure of the packaging container with water, particularly sterile water; and
collecting the water used for cleaning the closure of the packaging container after the cleaning process;
and wherein
before the treatment of the exterior of the packaging container with the agent at a temperature of less than 40°C, the agent is cooled in a heat exchanger with the help of the collected water as cooling medium.

6. Method according to any one of the preceding claims, in which the disinfectant is peracetic acid, and/or the agent is a peracetic acid solution.

7. Method according to any one of the preceding claims, in which the packaging container is a PET bottle.

8. Apparatus for the treatment of packaging containers, particularly PET bottles, comprising
a first rotor with receiving sites for the packaging containers;
a first device configured to treat the interior of the packaging containers at the receiving sites with a mixture consisting of water vapor and a disinfectant with a temperature of at least 90°C, particularly at least 95°C; and
a second device configured to treat, simultaneously with the treatment of the interior of the packaging containers, the exterior of the packaging containers with an agent comprising a disinfectant, at a temperature of less than 40°C, particularly with a temperature between 20°C and 30°C.

9. Apparatus according to claim 8, further comprising
a second rotor with receiving sites for the packaging containers, the second rotor being arranged downstream of the first rotor,
a third device configured to treat the exterior of the packaging container with the agent with a temperature of at least 40°C, particularly with a temperature between 40°C and 60°C.

10. Apparatus according to claim 9, further comprising a first heat exchanger configured to heat the agent before it is supplied to the third device for the treatment of the exterior of the packaging containers.

11. Apparatus according to any one of claims 8 to 10, further comprising a second heat exchanger configured to cool the agent before it is supplied to the second device for the treatment of the exterior of the packaging containers.

12. Apparatus according to any one of claims 8 to 11, further comprising a first rinser which is arranged downstream of the second rotor, and which is configured to clean the packaging containers with water, particularly sterile water, and a first collecting device which is configured to collect the water used for cleaning after the cleaning process and to supply it to the second heat exchanger as cooling medium.

13. Apparatus according to any one of claims 8 to 12, further comprising
a device configured to sterilize the closures of the packaging containers;
a second rinser which is arranged downstream of the device configured to sterilize the closures of the packaging containers, and which is configured to clean the closures of the packaging containers with water, particularly sterile water, and
a second collecting device configured to collect the water used for cleaning after the cleaning process and to supply it to the second heat exchanger as cooling medium.

## Revendications

1. Procédé de stérilisation de contenants d'emballage, comprenant les étapes suivantes :
alimenter et traiter l'intérieur d'un contenant d'emballage avec un mélange de vapeur d'eau et d'un désinfectant, avec une température d'au moins 90°C, notamment d'au moins 95°C ; et
simultanément alimenter et traiter l'extérieur du contenant d'emballage avec un agent, qui comprend un désinfectant, à une température inférieure à 40°C, notamment avec une température entre 20°C et 30°C.

2. Le procédé selon la revendication 1, d'après lequel l'agent est une solution de désinfectant, et comprenant, en outre, l'étape suivante : alimenter et traiter l'extérieur du contenant d'emballage avec l'agent avec une température d'au moins 40°C, notamment avec une température entre 40°C et 60°C, notamment après achèvement de l'étape consistant à alimenter et traiter l'intérieur du contenant d'emballage avec le mélange de vapeur d'eau et du désinfectant.

3. Le procédé selon la revendication 2, d'après lequel l'agent est véhiculé en circuit, et l'agent
a) est échauffé avant l'étape consistant à alimenter et traiter l'extérieur du contenant d'emballage avec l'agent avec une température d'au moins 40°C ; et
b) est refroidi avant l'étape consistant à alimenter et traiter l'extérieur du contenant d'emballage avec l'agent, à une température inférieure à 40°C.

4. Le procédé selon la revendication 3, comprenant en outre les étapes suivantes :
nettoyage du contenant d'emballage avec de l'eau, notamment de l'eau stérile, par une rinceuse, après l'étape consistant à alimenter et traiter l'extérieur du contenant d'emballage avec l'agent avec une température d'au moins 40°C, et
après le nettoyage, collecte de l'eau utilisée pour le nettoyage ;
procédé d'après lequel
avant l'étape consistant à alimenter et traiter l'extérieur du contenant d'emballage avec l'agent à une température inférieure à 40°C, l'agent est refroidi dans un échangeur de chaleur à l'aide de l'eau collectée, en guise de fluide de refroidissement.

5. Le procédé selon la revendication 3 ou la revendication 4, comprenant, en outre, les étapes suivantes :
stérilisation de la fermeture du contenant d'emballage; ensuite nettoyage de la fermeture du contenant d'emballage avec de l'eau, notamment de l'eau stérile ; et
collecte de l'eau utilisée pour le nettoyage de la fermeture du contenant d'emballage, après le nettoyage ;
procédé d'après lequel
avant l'étape consistant à alimenter et traiter l'extérieur du contenant d'emballage avec l'agent à une température inférieure à 40°C, l'agent est refroidi dans un échangeur de chaleur à l'aide de l'eau collectée, en guise de fluide de refroidissement.

6. Le procédé selon l'une des revendications précédentes, d'après lequel le désinfectant est de l'acide peracétique et/ou l'agent est une solution d'acide peracétique.

7. Le procédé selon l'une des revendications précédentes, d'après lequel le contenant d'emballage est une bouteille en PET.

8. Installation de traitement de contenants d'emballage, notamment des bouteilles en PET, comprenant un premier rotor avec des emplacements d'accueil pour les contenants d'emballage ;
un premier dispositif qui est conçu pour alimenter et traiter l'intérieur des contenants d'emballage au niveau des emplacements d'accueil, avec un mélange de vapeur d'eau et d'un désinfectant, avec une température d'au moins 90°C, notamment d'au moins 95°C ; et
un deuxième dispositif qui est conçu pour, simultanément avec l'étape consistant à alimenter et traiter l'intérieur des contenants d'emballage, alimenter et traiter l'extérieur des contenants d'emballage avec un agent, qui comprend un désinfectant, à une température inférieure à 40°C, notamment avec une température entre 20°C et 30°C.

9. L'installation selon la revendication 8, comprenant, en outre,
un deuxième rotor avec des emplacements d'accueil pour les contenants d'emballage, qui est monté en aval du premier rotor,
un troisième dispositif, qui est conçu pour alimenter et traiter l'extérieur des contenants d'emballage avec l'agent avec une température d'au moins 40°C, notamment avec une température entre 40°C et 60°C.

10. L'installation selon la revendication 9, comprenant, en outre, un premier échangeur de chaleur, qui est conçu pour échauffer l'agent, avant qu'il soit amené au troisième dispositif destiné à alimenter et traiter l'extérieur des contenants d'emballage.

11. L'installation selon l'une des revendications 8 à 10, comprenant, en outre, un deuxième échangeur de chaleur, qui est conçu pour refroidir l'agent avant qu'il ne soit acheminé au deuxième dispositif destiné à alimenter et traiter l'extérieur des contenants d'emballage.

12. L'installation selon l'une des revendications 8 à 11, comprenant, en outre, une première rinceuse, qui est montée en aval du deuxième rotor et est conçue pour nettoyer les contenants d'emballage avec de l'eau, notamment de l'eau stérile, et comprenant un premier dispositif de collecte, qui est conçu pour collecter, après le nettoyage, l'eau utilisée pour le nettoyage et l'amener au deuxième échangeur de chaleur, en tant que fluide de refroidissement.

13. L'installation selon l'une des revendications 8 à 12, comprenant, en outre,
un dispositif, qui est conçu pour stériliser les fermetures des contenants d'emballage ;
une deuxième rinceuse, qui est montée en aval du dispositif conçu pour stériliser les fermetures des contenants d'emballage, et qui est conçue pour nettoyer les fermetures des contenants d'emballage avec de l'eau, notamment de l'eau stérile, et
un deuxième dispositif de collecte, qui est conçu pour collecter, après le nettoyage, l'eau utilisée pour le nettoyage et l'amener au deuxième échangeur de chaleur, en tant que fluide de refroidissement.
